# EUROPEAN PATENT SPECIFICATION

(11) **EP 1 951 339 B1**
(45) Date of publication and mention of the grant of the patent: **04.08.2010**
(21) Application number: 06820812.3
(22) Date of filing: 26.10.2006
(51) Int. Cl.: A61M 1/36, A61F 7/00

(54) **APPARATUS FOR LOCAL ONCOLOGICAL TREATMENT**
GERÄT ZUR LOKALEN ONKOLOGISCHEN BEHANDLUNG
APPAREIL POUR UN TRAITEMENT ONCOLOGIQUE LOCAL

(30) Priority: 27.10.2005 IT BO20050655
(43) Date of publication of application: 06.08.2008
(73) Proprietor: Euromedical S.R.L., 25010 San Zeno Naviglio (IT)
(72) Inventor: FECONDINI, Luciano, I-40129 Bologna (IT); GIUBERTONI, Daniele, I-41037 Mirandola (IT); MUGNAI, Maurizio, I-41032 Cavezzo (IT); PALUMBO, Giuseppe, I-41100 Modena (IT); SALANI, Sergio, I-41037 Mirandola (IT)
(74) Representative: Cernuzzi, Daniele
(86) International application number: PCT/IB2006/003007
(87) International publication number: WO 2007/049134

(56) References cited:
- EP-A- 1 101 502
- WO-A-01/68031
- US-A1- 3 140 716
- US-A1- 4 177 816
- US-A1- 2005 080 373
- US-B1- 6 406 452

## Description

### TECHNICAL FIELD

The present invention relates to an apparatus for local oncological treatment.

### BACKGROUND ART

As is known, local oncological treatment is substantially of two types. In a first, blood circulation in a predetermined body region of the patient is isolated; the blood is then circulated outside the body; and, finally, a liquid chemotherapy drug is fed into the blood in the circuit outside the body. In the second type, performed after surgical removal of the tumour, the operated region is washed with a physiological solution, in which the liquid chemotherapy drug is diluted, by circulating the solution to and from the patient. Whichever the case - whether circulating a blood and liquid chemotherapy drug solution, or a physiological solution in which the liquid chemotherapy drug is diluted - the solution, before being administered to the patient, must be heated to a maximum temperature of 42-45°C : in the first case, to enhance the effectiveness of the liquid chemotherapy drug, and, in the second, to reduce the risk of relapse caused by the presence of active tumour cells. An apparatus is therefore required which, among other things, provides for determining an extremely precise temperature of the solution.

In addition to means for conducting the various fluids involved, and means for determining the temperature along the various conduits, currently marketed apparatuses also comprise a hot-plate heat exchanger, at which heat is yielded to the solution.

Apparatuses of the above type have numerous serious drawbacks.

Foremost of these is the long time - roughly forty minutes in known apparatuses - taken to bring the solution to be administered to the patient to a predetermined temperature, and which is mainly due to the high degree of thermal inertia typical of the hot plate of the heat exchanger. Obviously, the faster the predetermined temperature is reached, the sooner the treatment described above can be commenced, with obvious benefits to the patient. Another drawback of known apparatuses is that they fail to provide for rapidly varying heat exchange and, therefore, the temperature of the solution. The required solution temperature is obviously established by the operator according to the type of treatment, the type of liquid chemotherapy drug used, the patient's personal characteristics, and the type of surgery performed on the patient, and cannot be varied rapidly on account of the high degree of thermal inertia of the hot plate. Another important point to note is that the heat exchanger is installed in a frame containing most of the devices forming part of the apparatus, so that conduits are provided to conduct the solution from the exchanger to the patient and vice versa. Along the conduits, heat is obviously lost in direct proportion to the length of the conduits, so that the temperature of the solution administered to the patient is lower than that of the solution leaving the heat exchanger, whereas, as stated, fast intervention and extremely precise temperature of the solution administered to the patient are essential for ensuring the effectiveness of the treatment.

### DISCLOSURE OF INVENTION

It is an object of the present invention to provide an apparatus for local oncological treatment, designed to eliminate the aforementioned drawbacks, and which, in particular, provides for rapidly heating the solution to be administered to the patient to a predetermined temperature, comprises an easy-to-handle heat exchanger that can be moved closer to the patient to greatly reduce heat dispersion, has an extensive heat-exchange surface, and provides for rapidly varying the desired solution temperature.

According to the present invention, there is provided an apparatus for local oncological treatment, comprising :
a first conduit for conducting a first liquid to and from a body region of a patient;
a heat exchanger installed along said first conduit;
a source of a second liquid defined by a chemotherapy drug;
a second conduit originating from said source and connected to said first conduit upstream from said heat exchanger;
a member for feeding a third liquid, at a given temperature, to said heat exchanger to produce, inside the heat exchanger, heat transfer to said first liquid;
characterized in that said heat exchanger is a shell-and-tube exchanger.

### BRIEF DESCRIPTION OF THE DRAWINGS

A preferred embodiment of the present invention will be described with reference to the accompanying drawings, in which:
Figure 1 shows a block diagram of an apparatus for local oncological treatment, for use in a first type of treatment;
Figure 2 shows a block diagram of an apparatus for local oncological treatment, for use in a second type of treatment;
Figure 3 shows a larger-scale section of a member of the Figure 1 and 2 apparatuses.

### BEST MODE FOR CARRYING OUT THE INVENTION

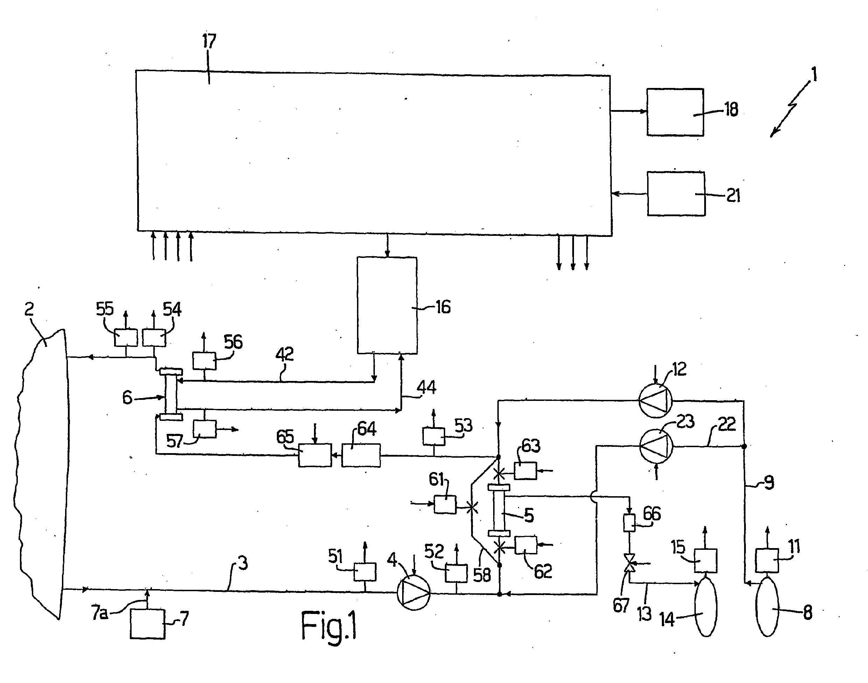

Number 1 in Figure 1 indicates as a whole an apparatus for local oncological treatment. Once the blood circulation in a predetermined body region of the patient 2 is isolated, apparatus 1 provides for circulating the blood outside the body, and for feeding a liquid chemotherapy drug into the blood circulating outside the body.

Apparatus 1 comprises:
a conduit 3 for conducting the patient's blood to and from said body region;
a first pump 4 installed along conduit 3;
a blood filter 5 installed along conduit 3, downstream from pump 4;
a heat exchanger 6 installed along conduit 3, downstream from blood filter 5;
a source 7 of a liquid chemotherapy drug, which is fed by a conduit 7a into conduit 3, upstream from pump 4;
a bag 8 of physiological solution for supplementing the blood downstream from blood filter 5;
a conduit 9 originating from bag 8 and connected to conduit 3, between blood filter 5 and heat exchanger 6;
means 11 for determining the weight of the supplementing physiological solution inside bag 8;
a second pump 12 installed along conduit 9;
a conduit 13 originating from blood filter 5 and which conducts the ultrafiltrate from blood filter 5 to a container 14 (shown schematically in Figure 1);
means 15 for determining the weight of the ultrafiltrate in container 14;
a member 16 for heating a liquid, e.g. water, to a predetermined temperature; and
an electronic central control unit 17 for controlling apparatus 1.

A display 18, for displaying the quantities involved, and a data entry block 21 are connected to electronic central control unit 17. Apparatus 1 also comprises a conduit 22, which originates from conduit 9, feeds a predetermined amount of supplementing physiological solution into conduit 3, between pump 4 and blood filter 5, and is fitted with a pump 23.

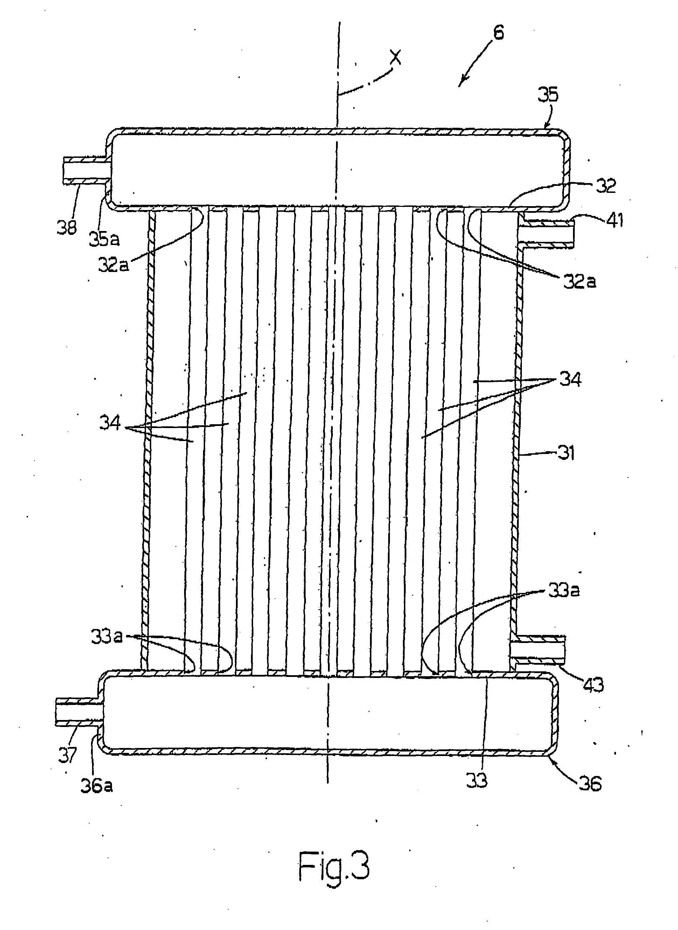

With reference to Figure 3, heat exchanger 6 is a shell-and-tube type. More specifically, heat exchanger 6 comprises a hollow cylindrical central body 31 closed at its axial ends by respective walls 32 and 33, and in which are installed a number of preferably equally spaced tubes 34 with longitudinal axes parallel to one another and to the longitudinal axis X of body 31. In walls 32, 33 are formed respective numbers of through holes 32a, 33a, in which the corresponding axial ends of tubes 34 are fixed, e.g. glued, to form a hydraulic seal between the inside of tubes 34 and the inside of body 31. At the axial ends of body 31, heat exchanger 6 has a hollow cylindrical body 35, of which wall 32 forms the bottom wall; and a hollow cylindrical body 36, of which wall 33 forms the top wall. The lateral wall 36a of body 36 has an inlet 37, to which is connected the free end of the portion of conduit 3 extending between blood filter 5 and heat exchanger 6. The lateral wall 35a of body 35 has an outlet 38, to which is connected the free end of the portion of conduit 3 extending between heat exchanger 6 and the body region of patient 2. Close to body 35, body 31 has an inlet 41, to which is connected the free end of a conduit 42 for conducting the heated liquid from member 16. Close to body 36, body 31 has an outlet 43, to which is connected the free end of a conduit 44 for feeding the liquid back to member 16. Walls 32 and 33 may be made of rigid plastic material, or of semirigid or flexible plastic material, such as polyurethane, in which to embed the axial ends of tubes 34.

A number of sensors are installed along the conduits described above, to determine the temperature and pressure along the conduits, and to supply this information to electronic central control unit 17.

More specifically, apparatus 1 comprises:
a pressure sensor 51 installed along conduit 3, upstream from pump 4;
a pressure sensor 52 installed along conduit 3, immediately downstream from pump 4;
a pressure sensor 53 installed along conduit 3, between blood filter 5 and heat exchanger 6 and downstream from the connection to conduit 9;
two temperature sensors 54 and 55 installed in series along conduit 3, immediately downstream from heat exchanger 6;
a temperature sensor 56 installed along conduit 42; and
a temperature sensor 57 installed along conduit 44.

Apparatus 1 also comprises:
a bypass conduit 58 installed along conduit 3, in parallel with blood filter 5;
a clamp 61 installed along bypass conduit 58 to open or close circulation along conduit 58;
a clamp 62 installed along conduit 3, immediately upstream from blood filter 5 and downstream from where bypass conduit 58 branches off from conduit 3, to open or close circulation to blood filter 5;
a clamp 63 installed along conduit 3, immediately downstream from blood filter 5 and upstream from connection of bypass conduit 58 to conduit 3, to open or close circulation from blood filter 5;
a drip 64 installed along conduit 3, immediately downstream from sensor 53;
a clamp 65 installed along conduit 3, immediately downstream from drip 64;
a blood loss detector 66 installed along conduit 13; and
an ultrafiltrate flow regulator 67 installed along conduit 13, downstream from detector 66.

Clamps 61, 62, 63, 65 and regulator 67 may be operated manually or controlled by central control unit 17.

In actual use, the operating data of apparatus 1 - substantially, the temperature to which the blood is to be heated in heat exchanger 6, and the amount of ultrafiltrate to be removed from the blood in blood filter 5 - is set on the basis of the patient's personal characteristics, the type of surgery performed on the patient, and the body region in which blood circulation has been isolated. Once the above data is set, the liquid is heated by member 16 and temperature-controlled by sensors 56 and 57. Once the liquid from member 16 reaches a predetermined temperature, the blood is circulated along conduit 3, and the temperature of the blood from heat exchanger 6 is controlled by sensors 54 and 55. The double temperature control by sensors 54 and 55 enhances the safety of apparatus 1. At this point, electronic central control unit 17 compares the temperature readings of sensors 54 and 55 with the temperature setting of block 21, and accordingly commands member 16 to regulate the temperature of the liquid from member 16 and therefore heat exchange with the blood inside heat exchanger 6. To improve performance of heat exchanger 6, a temperature sensor may be installed along conduit 3, at the outlet from the body region.

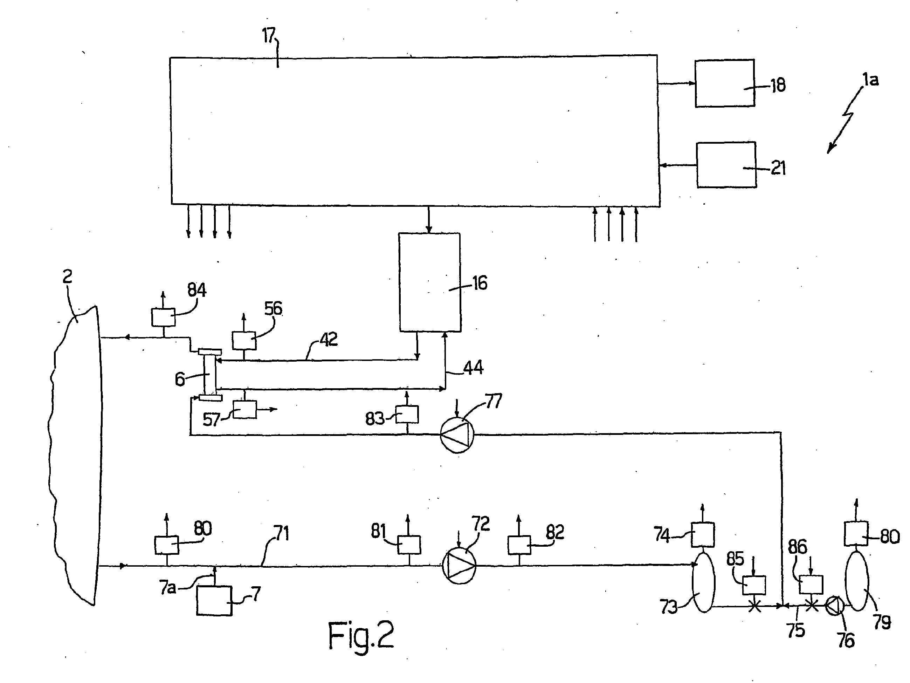

With reference to Figure 2, number 1a indicates an apparatus for washing the operated body region with a water and liquid chemotherapy drug solution, by circulating the solution to and from patient 2.

Apparatus 1a comprises:
a conduit 71 for conducting to and from patient 2 a quantity of physiological infusion solution, in which a quantity of liquid chemotherapy drug is diluted;
a first pump 72 installed along conduit 71;
a container 73 (shown schematically) installed along conduit 71;
means 74 for determining the weight of the liquid in container 73;
said source 7 of a liquid chemotherapy drug which is fed by conduit 7a into conduit 71, upstream from pump 72;
a bag 79 of physiological solution;
a conduit 75 originating from bag 79 and connected to conduit 71, downstream from container 73;
means 80 for determining the weight of bag 79;
a pump 76 installed along conduit 75;
a pump 77 installed along conduit 71, downstream from the connection to conduit 75;
said heat exchanger 6 installed along conduit 71, downstream from pump 77;
said member 16 for heating a liquid, e.g. water, to a predetermined temperature;
said electronic central control unit 17 for controlling apparatus 1a;
said display 18;
said data entry block 21; and
said conduits 42 and 44.

Apparatus 1a also has a number of sensors installed along the conduits described above, to determine the temperature and pressure along the conduits, and to supply this information to electronic central control unit 17.

More specifically, apparatus 1a comprises:
a temperature sensor 80 installed along conduit 71, immediately downstream from the outlet of the body region;
a pressure sensor 81 installed along conduit 71, upstream from pump 72;
a pressure sensor 82 installed along conduit 71, immediately downstream from pump 72;
a pressure sensor 83 installed along conduit 71, immediately downstream from pump 77;
a temperature sensor 84 installed along conduit 71, immediately downstream from heat exchanger 6;
said sensor 56 installed along conduit 42; and
said sensor 57 installed along conduit 44.

Finally, apparatus 1a comprises a clamp 85 installed along conduit 71, between container 73 and the connection to conduit 75; and a clamp 86 installed along conduit 75.

In actual use, a space in the body region from which the tumour has been removed is fed by conduit 71 with a quantity of physiological solution, in which a liquid chemotherapy drug is diluted, and which of course is first heated to a predetermined temperature by heat exchanger 6. The flow rates, temperatures, and pressures involved are controlled by central control unit 17 in the same way as for apparatus 1 in Figure 1. At the start of treatment, container 73 is filled with a predetermined quantity of solution from bag 79, and, after closing conduit 75 by means of clamp 86, the solution, in which the liquid drug is diluted, is circulated along conduit 71.

The numerous advantages of the present invention will be clear from the foregoing description.

In particular, an apparatus is provided which, with only minor alterations, can be used for two different types of treatment. Moreover, using a shell-and-tube heat exchanger provides for fast, effective heat exchange. In laboratory tests conducted by the Applicant, circulation of the liquid (blood diluted with a liquid chemotherapy drug, or solution in which a liquid chemotherapy drug is diluted) into the patient was found to take only a few minutes. The speed with which the predetermined temperature is reached is mainly due to the extensive exchange surface of the heat exchanger. All of which provides for fast, effective treatment immediately following surgery. Another important point to note is that the heat exchanger used is an easy-to-handle item, can be located outside the frame housing the various component parts of the apparatus, and, in particular, can be located close to the patient to reduce the length of the conduit portion between the exchanger and the patient, and therefore greatly reduce heat dispersion. In fact, the temperature of the liquid infused into the patient differs hardly at all (by a barely detectable amount) from the temperature of the liquid issuing from the heat exchanger. Another point to note is that, with the exception of the pumps, central control unit 17, and member 16, all the other components are disposable, thus ensuring a high degree of user safety. Finally, the apparatus comprises sensors and detectors for continuously monitoring the quantities involved, and so enabling adjustment of certain quantities and termination of treatment in the event of anomalies.

Clearly, changes may be made to the apparatus as described and illustrated herein without, however, departing from the scope of the present invention.

In particular, in heat exchanger 6, the heating liquid may obviously circulate along tubes 34, and the liquid for infusion into the patient may circulate inside body 31.

## Claims

1. An apparatus for local oncological treatment, comprising :
a first conduit (3 or 71) for conducting a first liquid to and from a body region of a patient (2);
a heat exchanger (6) installed along said first conduit (3 or 71);
a source (7) of a second liquid defined by a chemotherapy drug;
a second conduit (7a) originating from said source (7) and connected to said first conduit (3 or 71) upstream from said heat exchanger (6);
a member (16) for feeding a third liquid, at a given temperature, to said heat exchanger (6) to produce, inside the heat exchanger, heat transfer to said first liquid;
**characterized in that** said heat exchanger (6) is a shell-and-tube exchanger.

2. An apparatus as claimed in Claim 1, **characterized in that** said heat exchanger (6) comprises:
a hollow cylindrical central body (31) closed at its axial ends by respective walls (32 and 33);
a number of tubes (34) installed inside said central body (31) and having longitudinal axes parallel to one another and to the longitudinal axis (X) of said central body (31);
respective numbers of through holes (32a and 33a) formed in said walls (32 and 33), and in which the corresponding axial ends of said tubes (34) are fixed to form a hydraulic seal between the inside of said tubes (34) and the inside of said central body (31);
two hollow end bodies (35 and 36) defined at the axial ends of said central body (31) and communicating with each other via said tubes (34);
one liquid selected from said first liquid and said third liquid circulating along said tubes (34) and in said end bodies (35 and 36), and the other liquid selected from said first liquid and said third liquid circulating inside said central body (31).

3. An apparatus as claimed in Claim 2, **characterized in that** an inlet (41) and an outlet (43) are formed in said central body (31), and an outlet and an inlet (38 and 37) are formed respectively in said end bodies (35 and 36) to circulate said liquid.

4. An apparatus as claimed in at least one of the foregoing Claims, **characterized by** comprising a first temperature sensor (54 or 84) installed along said first conduit (3 or 71), downstream from said heat exchanger (6), and a second temperature sensor (56) installed along a third conduit (42) for conducting said third liquid from said member (16) to said heat exchanger (6).

5. An apparatus as claimed in at least one of the foregoing Claims, **characterized in that** the heat exchanger is located outside a frame housing the various component parts of the apparatus, so as to be possibly located close to a patient.

6. An apparatus as claimed in Claim 4 and/or Claim 5, **characterized by** comprising at least one pump (4, 72 and/or 77) installed along said first conduit (3 or 71).

7. An apparatus as claimed in Claim 6, **characterized by** comprising a bag (8) containing a physiological solution; and a conduit (9 or 22) for feeding said solution into said first conduit (3).

8. An apparatus as claimed in Claim 7, **characterized by** comprising means (11) for determining the amount of said solution in said bag (8).

9. An apparatus as claimed in Claims 4 to 7, **characterized by** comprising an electronic central control unit (17) for controlling the various component parts of said apparatus on the basis of the findings of said sensors.

10. An apparatus as claimed in Claim 9, **characterized in that** a display (18), for displaying the various quantities detected, and a data entry block (21) are connected to said central control unit.

11. An apparatus as claimed in at least one of the foregoing Claims, **characterized in that** said first liquid is defined by blood diluted with a liquid chemotherapy drug.

12. An apparatus as claimed in Claim 11, **characterized by** comprising a blood filter (5) installed along the first conduit (3), upstream from said heat exchanger (6).

13. An apparatus as claimed in at least one of Claims 1 to 10, **characterized in that** said first liquid is defined by a physiological solution, in which a liquid chemotherapy drug is diluted.

## Patentansprüche

1. Gerät zur lokalen onkologischen Behandlung, umfassend:
eine erste Leitung (3 oder 71) zum Leiten einer ersten Flüssigkeit zu und von einer Körperregion eines Patienten (2);
einen Wärmeaustauscher (6), der entlang der ersten Leitung (3 oder 71) montiert ist;
eine Quelle (7) für eine zweite, durch einen chemotherapeutischen Arzneistoff definierte Flüssigkeit;
eine zweite Leitung (7a), die an der Quelle (7) beginnt und an die erste Leitung (3 oder 71) stromaufwärts von dem Wärmeaustauscher (6) angeschlossen ist;
ein Element (16) zum Zuführen einer dritten Flüssigkeit, bei einer gegebenen Temperatur, in den Wärmeaustauscher (6), um im Inneren des Wärmeaustauschers (6) eine Wärmeübertragung auf die erste Flüssigkeit herbeizuführen;
**dadurch gekennzeichnet, dass** der Wärmeaustauscher (6) ein Schalen-und-Röhren-Austauscher ist.

2. Gerät nach Anspruch 1, **dadurch gekennzeichnet, dass** der Wärmeaustauscher (6) folgendes umfasst:
einen hohlen, zylindrischen Zentralkörper (31), der an seinen axialen Enden jeweils durch die Wände (32 und 33) geschlossen ist;
eine Anzahl von Röhren (34), die im Inneren des Zentralkörpers montiert sind und mit zueinander und zu der Längsachse (X) des Zentralkörpers (31) parallelen Längsachsen;
entsprechende Anzahlen von Durchgangsbohrungen (32a und 33a), die in den Wänden (32 und 33) ausgebildet sind und in denen die entsprechenden axialen Enden der Röhren (34) fixiert sind, um eine hydraulische Dichtung zwischen dem Inneren der Röhren (34) und dem Inneren des Zentralkörpers (31) zu bilden;
zwei hohle Endkörper (35 und 36), die an den axialen Enden des Zentralkörpers (31) definiert sind und miteinander über die Röhren (34) kommunizieren;
eine Flüssigkeit, die aus der ersten Flüssigkeit und der dritten Flüssigkeit ausgewählt ist, die entlang der Röhren (34) und in den Endkörpern (35 und 36) zirkulieren, wobei die andere Flüssigkeit, die aus der ersten Flüssigkeit und der dritten Flüssigkeit ausgewählt ist, im Inneren des Zentralkörpers (31) zirkuliert.

3. Gerät nach Anspruch 2, **dadurch gekennzeichnet, dass** ein Einlass (41) und ein Auslass (43) in dem Zentralkörper (31) ausgebildet sind, und ein Auslass bzw. ein Einlass (38 bzw. 37) in den Endkörpern (35 und 36) ausgebildet sind, um die Flüssigkeit zu zirkulieren.

4. Gerät nach mindestens einem der vorhergehenden Ansprüche, **dadurch gekennzeichnet, dass** es einen ersten Temperatursensor (54 oder 84), der entlang der ersten Leitung (3 oder 71), stromabwärts von dem Wärmeaustauscher (6) montiert ist, und einen zweiten Temperatursensor (56), der entlang einer dritten Leitung (42) montiert ist, zum Leiten der dritten Flüssigkeit von dem Element (16) zu dem Wärmeaustauscher (6) umfasst.

5. Gerät nach mindestens einem der vorhergehenden Ansprüche, **dadurch gekennzeichnet, dass** der Wärmeaustauscher außerhalb eines Rahmens angeordnet ist, der die verschiedenen Bauteile des Geräts einhaust, so dass die Möglichkeit besteht, dass er nahe an einem Patienten angeordnet ist.

6. Gerät nach mindestens Anspruch 4 und/oder Anspruch 5, **dadurch gekennzeichnet, dass** es mindestens eine Pumpe (4, 72 und/oder 77) umfasst, die entlang der ersten Leitung (3 oder 71) montiert ist.

7. Gerät nach Anspruch 6, **dadurch gekennzeichnet, dass** es eine Beutel (8), der eine physiologische Lösung enthält; und eine Leitung (9 oder 22) zum Zuführen der Lösung in die erste Leitung (3) umfasst.

8. Gerät nach Anspruch 7, **dadurch gekennzeichnet, dass** es Mittel (11) zum Bestimmen der Menge der Lösung in der Beutel (8) umfasst.

9. Gerät nach den Ansprüchen 4 bis 7, **dadurch gekennzeichnet, dass** es eine elektronische zentrale Steuereinheit (17) zum Steuern der verschiedenen Bauteile des Geräts auf der Grundlage der Befunde der Sensoren umfasst.

10. Gerät nach Anspruch 9, **dadurch gekennzeichnet, dass** eine Anzeige (18) zum Anzeigen der verschiedenen nachgewiesenen Mengen und ein Dateneingabeblock (21) an die zentrale Steuereinheit angeschlossen sind.

11. Gerät nach mindestens einem der vorhergehenden Ansprüche, **dadurch gekennzeichnet, dass** die erste Flüssigkeit durch Blut definiert ist, das mit einem flüssigen chemotherapeutischen Arzneistoff verdünnt ist.

12. Gerät nach Anspruch 11, **dadurch gekennzeichnet, dass** es ein entlang der ersten Leitung (3) stromaufwärts von dem ersten Wärmeaustauscher (6) montiertes Blutfilter (5) umfasst.

13. Gerät nach mindestens einem der vorhergehenden Ansprüche 1 bis 10, **dadurch gekennzeichnet, dass** die erste Flüssigkeit durch eine physiologische Lösung definiert ist, in der ein flüssiger chemotherapeutischer Arzneistoff verdünnt ist.

## Revendications

1. Appareil destiné à un traitement oncologique local, comprenant :
un premier conduit (3 ou 71) destiné à faire circuler un premier liquide vers et depuis une région du corps d'un patient (2) ;
un échangeur thermique (6) installé le long dudit premier conduit (3 ou 71) ;
une source (7) d'un second liquide défini par un médicament chimiothérapique ;
un second conduit (7a) qui provient de ladite source (7) et relié audit premier conduit (3 ou 71) en amont dudit échangeur thermique (6) ;
un élément (16) destiné à alimenter un troisième liquide, à une température donnée, audit échangeur thermique (6) afin de produire, à l'intérieur de l'échangeur thermique, un transfert de chaleur vers ledit premier liquide ;
**caractérisé en ce que** ledit échangeur thermique (6) est un échangeur multitubulaires.

2. Appareil tel que revendiqué dans la revendication 1, **caractérisé en ce que** ledit échangeur thermique (6) comprend :
un corps central cylindrique creux (31) fermé au niveau de ses extrémités axiales par des parois respectives (32 et 33) ;
un nombre de tubes (34) installés à l'intérieur dudit corps central (31) et ayant des axes longitudinaux parallèles entre eux et parallèles à l'axe longitudinal (X) dudit corps central (31) ;
des nombres respectifs de trous traversants (32a et 33a) formés dans lesdites parois (32 et 33), et dans lesquels les extrémités axiales correspondantes desdits tubes (34) sont fixées afin de former un joint hydraulique entre l'intérieur desdits tubes (34) et l'intérieur dudit corps central (31) ;
deux corps d'extrémités creux (35 et 36) définis au niveau des extrémités axiales dudit corps central (31) et communiquant entre eux par l'intermédiaire desdits tubes (34) ;
un liquide sélectionné dudit premier liquide et dudit troisième liquide circulant le long desdits tubes (34) et dans lesdits corps (35 et 36), et l'autre liquide sélectionné dudit premier liquide et dudit troisième liquide circulant à l'intérieur dudit corps central (31).

3. Appareil tel que revendiqué dans la revendication 2, **caractérisé en ce qu'**une entrée (41) et une sortie (43) sont formées dans ledit corps central (31), et une sortie et une entrée (38 et 37) sont formées respectivement dans lesdits corps d'extrémités (35 et 36) afin de faire circuler ledit liquide.

4. Appareil tel que revendiqué dans l'une des revendications précédentes au moins, **caractérisé par** le fait de comprendre un premier capteur de température (54 ou 84) installé le long dudit premier conduit (3 ou 71), en aval dudit échangeur thermique (6), et un second capteur de température (56) installé le long d'un troisième conduit (42) destiné à faire circuler ledit troisième liquide dudit élément (16) audit échangeur thermique (6).

5. Appareil tel que revendiqué dans l'une des revendications précédentes au moins, **caractérisé en ce que** l'échangeur thermique est placé à l'extérieur d'un châssis accueillant les diverses parties constitutives de l'appareil, de manière à pouvoir être éventuellement placé à proximité d'un patient.

6. Appareil tel que revendiqué dans la revendication 4 et/ou la revendication 5, **caractérisé par** le fait de comprendre au moins une pompe (4, 72 et/ou 77) installée le long dudit premier conduit (3 ou 71).

7. Appareil tel que revendiqué dans la revendication 6, **caractérisé par** le fait de comprendre une poche (8) contenant une solution physiologique ; et un conduit (9 ou 22) pour alimenter ladite solution dans ledit premier conduit (3).

8. Appareil tel que revendiqué dans la revendication 7, **caractérisé par** le fait de comprendre un moyen (11) destiné à déterminer la quantité de ladite solution se trouvant dans ladite poche (8).

9. Appareil tel que revendiqué dans les revendications 4 à 7, **caractérisé par** le fait de comprendre une unité de commande centrale électronique (17) destinée à commander les diverses parties constitutives dudit appareil sur la base des détections desdits capteurs.

10. Appareil tel que revendiqué dans la revendication 9, **caractérisé en ce qu'**un écran (18), destiné à afficher les diverses quantités détectées, et un pavé d'entrée de données (21) sont connectés à ladite unité de commande centrale.

11. Appareil tel que revendiqué dans l'une des revendications précédentes au moins, **caractérisé en ce que** ledit premier liquide est défini par du sang dilué avec un médicament chimiothérapique liquide.

12. Appareil tel que revendiqué dans la revendication 11, **caractérisé par** le fait de comprendre un filtre sanguin (5) installé le long du premier conduit (3), en amont dudit échangeur thermique (6).

13. Appareil tel que revendiqué dans l'une des revendications 1 à 10 au moins, **caractérisé en ce que** ledit premier liquide est défini par une solution physiologique, dans laquelle un médicament chimiothérapique liquide est dilué.
